(19) Europäisches Patentamt
European Patent Office
Office européen des brevets

(11) Veröffentlichungsnummer : **0 172 138**
**B1**

(12) # EUROPÄISCHE PATENTSCHRIFT

(45) Veröffentlichungstag der Patentschrift : **15.06.88**

(51) Int. Cl.⁴ : **C 07 D405/04**, C 08 K 5/34

(21) Anmeldenummer : **85810350.0**

(22) Anmeldetag : **29.07.85**

(54) **N-Piperidyl-tetrahydro-1,4-oxazin-2-one als Lichtschutzmittel.**

(30) Priorität : **02.08.84 US 637240**

(43) Veröffentlichungstag der Anmeldung : **19.02.86 Patentblatt 86/08**

(45) Bekanntmachung des Hinweises auf die Patenterteilung : **15.06.88 Patentblatt 88/24**

(84) Benannte Vertragsstaaten : **DE FR GB IT**

(56) Entgegenhaltungen :
**EP-A- 0 019 578**
**EP-A- 0 034 805**
**DE-A- 2 801 470**
**US-A- 4 298 737**

(73) Patentinhaber : **CIBA-GEIGY AG**
**Klybeckstrasse 141**
**CH-4002 Basel (CH)**

(72) Erfinder : **Malherbe, Roger F., Dr.**
**Bleichestrasse 7**
**CH-4058 Basel (CH)**
Erfinder : **Ackerman, Michael H., Dr.**
**216 Morris Drive**
**East Meadow, N.Y. 11554 (US)**

**EP 0 172 138 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Verbindungen, die sowohl eine Tetrahydro-1,4-oxazin-2-on-Gruppe als auch eine sterisch gehinderte substituierte 4-Piperidinyl-Gruppe enthalten und die als Lichtschutzmittel für organische Materialien verwendet werden können. Die Erfindung betrifft auch stabilisierte Gemische enthaltend diese Verbindungen.

Sterisch gehinderte Amine mit einer 2,2,6,6,-tetrasubstituierten Piperidinstruktur sind seit längerem als wirkungsvolle Lichtschutzmittel für organische Materialien bekannt und haben beträchtlichen wirtschaftlichen Erfolg gefunden. Solche Lichtschutzmittel sind beispielsweise beschrieben von Heller und Blattmann/Pure & Appl. Chem. 36 (1973), 141-161.

In der UP-PS 3,684,765 sind 4-Aminopiperidinderivate der Formel

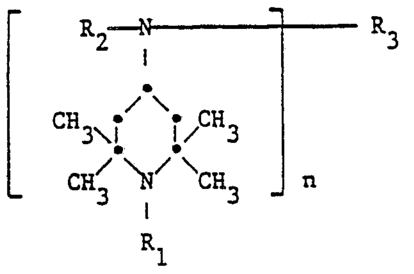

beschrieben, worin n 1, 2 oder 3 ist, $R_1$ H oder Acyl bedeutet $R_2$ H, Alkyl oder Aryl bedeutet und $R_3$ Wasserstoff oder einen ein-, zwei- oder dreiwertigen Acylrest bedeutet.

Verbindungen, worin $R_2$ und $R_3$ zusammen bestimmte heterocyclische Ringe bilden, sind in den US-PS 4,033,928, 4,298,737, 4,309,546 und 4,472,547 beschrieben.

Keine dieser Patentschriften beschreibt Verbindungen oder legt Verbindungen nahe, in denen ein Tetrahydro-1,4-oxazin-2-on-Rest an eine sterisch gehinderte Piperidingruppe gebunden ist. Solche Verbindungen besitzen hervorragende Stabilisatorwirkung verbunden mit guter Löslichkeit und guter thermischer und hydrolytischer Beständigkeit.

Die vorliegende Erfindung betrifft Verbindungen der Formel I

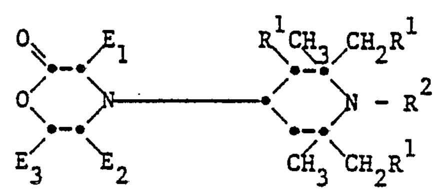

(I)

worin

$E_1$ Wasserstoff, $C_1$—$C_{16}$-Alkyl oder Phenyl bedeutet,

$E_2$ Wasserstoff oder Methyl bedeutet,

$E_3$ Wasserstoff, $C_1$—$C_4$-Alkyl oder Phenyl bedeutet,

$R^1$ Wasserstoff oder $C_1$—$C_5$-Alkyl bedeutet und

$R^2$ Wasserstoff, Sauerstoff, Hydroxyl, $C_1$—$C_{12}$-Alkyl, $C_3$—$C_8$-Alkenyl, Propargyl, Benzyl, Cyan, $C_2$—$C_4$-Hydroxyalkyl, $C_2$—$C_{10}$-Alkanoyl, $C_3$—$C_4$-Alkenoyl, Benzoyl, $C_1$—$C_8$-Alkoxy, $C_2$—$C_{12}$-Alkanoyloxy, $C_3$—$C_4$-Alkenoyloxy oder Benzoyloxy bedeutet.

$E_1$ als Alkyl kann z. B. Methyl, Ethyl, Isopropyl, n-Butyl, n-Amyl, 2- Ethylhexyl, n-Decyl, n-Dodecyl oder n-Tetradecyl sein. $E_2$ ist vorzugsweise Wasserstoff.

$E_3$ als Alkyl kann z. B. Methyl, Ethyl, n-Propyl oder n-Butyl sein. Vorzugsweise ist $E_3$ Wasserstoff oder Methyl.

$R^1$ als Alkyl kann z. B. Methyl, Ethyl, n-Butyl oder n-Amyl sein. Vor zugsweise ist $R^1$ Wasserstoff.

$R^2$ als Alkyl kann z. B. Methyl, Ethyl, Isopropyl, sec.Butyl, n-Amyl, 2-Ethylhexyl, n-Decyl oder n-Dodecyl sein.

$R^2$ als Alkenyl kann z. B. Allyl, Butenyl, Crotyl oder Octenyl sein. $R^2$ als Hydroxyalkyl kann z. B. 2-Hydroxyethyl, 2-Hydroxypropyl oder 2-Hydroxybutyl sein.

$R^2$ als Alkanoyl kann z. B. Acetyl, Propionyl, Butyryl, Valeroyl, Caproyl, Capryloyl oder Decanoyl sein. $R^2$ als Alkenoyl kann z. B. Acryloyl, Methacryloyl oder Crotonyl sein.

$R^2$ als Alkoxy kann z. B. Methoxy, Ethoxy, Isopropoxy, n-Butoxy, Hexyloxy oder Octyloxy sein. $R^2$ als Alkanoyloxy kann z. B. Acetoxy, Propionyloxy, Butyryloxy, Valeroyloxy, Octanoyloxy oder Decanoyloxy sein. $R^2$ als Alkenoyloxy kann z. B. Acryloyloxy, Methacryloyloxy oder Crotonyloxy sein.

Vorzugsweise ist $R^2$ Wasserstoff, Sauerstoff, Hydroxyl, $C_1$—$C_4$-Alkyl, Allyl, 2-Hydroxyethyl, Acetyl, Propargyl, Benzyl, Cyan, Benzoyl oder Benzoyloxy, insbesondere Wasserstoff.

Zur Herstellung der Verbindungen der Formel I können zwei Wege beschritten werden, die im folgenden Als A und B skizziert sind.

(A) Umsetzung eines Glycinesters mit einem Oxiran :

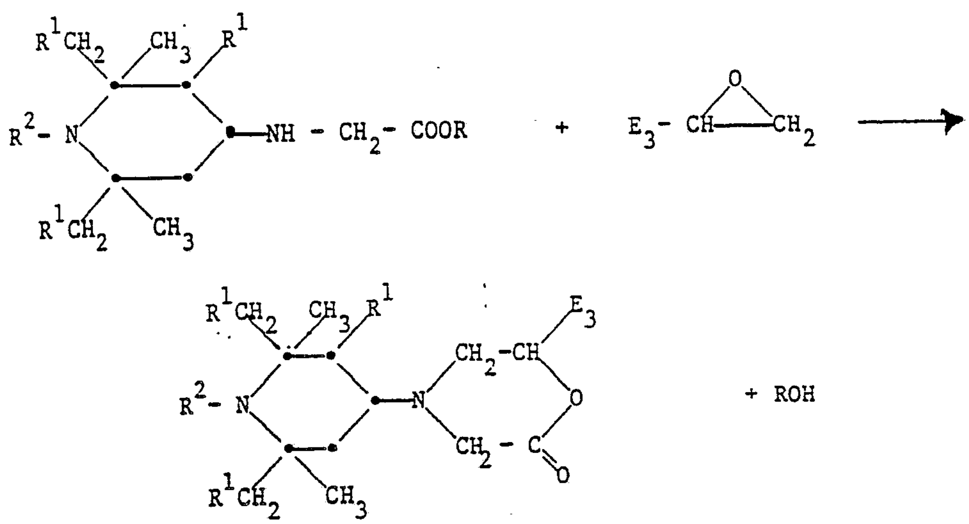

(R = Alkyl)

(B) Umsetzung eines 2-Aminoalkanols mit einem α-Halogencarbonsäureester in Gegenwart einer Base :

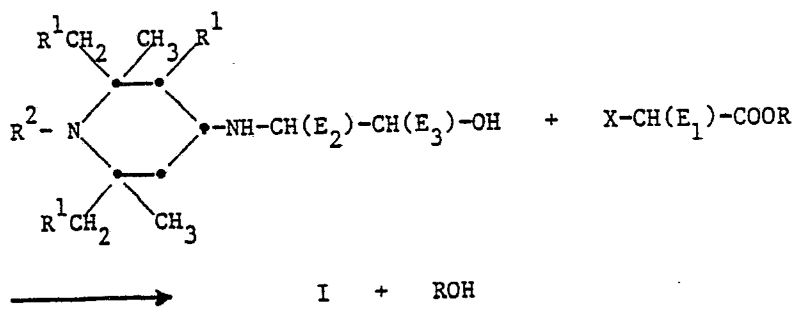

(X = Cl oder Br)

Bevorzugt geschieht die Herstellung nach Route B.

Die hierfür benötigten N-Piperidinyl-aminoalkohole sind einfach erhältlich durch reduktive Aminierung von Triacetonamin oder einem homologen 4-Ketopiperidin mit dem entsprechenden Aminoalkohol $H_2N—CH(E_2)—CH(E_3)—OH$. Solche Reaktionen sind beschrieben in der US-PS 3,684,765. Alternativ können diese Zwischenprodukte auch durch Hydroxyalkylierung der entsprechenden 4-Aminopiperidine hergestellt werden, wie dies in der US-PS 4,166,813 beschrieben ist.

Die Verbindungen der Formel I sind wirkungsvolle Lichtschutzmittel für organische Materialien, insbesondere für organische Polymere. Beispiele für Polymere, die mit diesen Verbindungen stabilisiert werden können, sind :

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und

einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen-/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannter Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrol-polymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexa-methylenterephthalamid- Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimid-azole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäure-estern ableiten wie z. B. von Epoxyacrylaten, Urethan-acrylaten oder Polyester-acrylaten.

24. Alkydharze, Polyesterharze und Acrylatharze, die mit Melamin-harzen, Harnstoffharzen, Polyisocyanaten, oder Epoxidharzen vernetzt sind.

25. Vernetzte Epoxidharze, die sich von Polyepoxiden ableiten, z. B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

Von besonderer Bedeutung ist die Stabilisierung von Polyolefinen, Styrol-Polymeren, Polyamiden und Polyurethanen.

Die Stabilisatoren werden den Polymeren in einer Menge von 0,05 bis 5 Gew.-%, vorzugsweise von 0,1 bis 2,5 Gew.-%, zugegeben. Die Zugabe kann vor oder nach der Polymerisation geschehen, beispielsweise durch Einmischen der Stabilisatoren und gegebenenfalls weiterer Additive in die Schmelze nach den in der Technik üblichen Methoden, vor oder während der Formgebung, oder durch Zusatz in gelöster Form zu einer Lösung oder oder Dispersion des Polymeren.

Die Stabilisatoren können den Polymeren auch in Form eines Masterbatches zugesetzt werden, worin die Stabilisatoren in einer Menge von 2,5 bis 25 Gew.-% vorliegen können.

Die Verbindungen der Formel I können in Kombination mit anderen Lichtschutzmitteln oder mit sonstigen Stabilisatoren wie z. B. Antioxydantien, Metalldesaktivatoren, thermischen Stabilisatoren und Costabilisatoren verwendet werden. Weitere Additive, die gemeinsam mit den Verbindungen der Formel I zugesetzt werden können, sind z. B. Flammschutzmittel, Antistatica, Weichmacher, Gleitmittel, Füllstoffe, Verstärkungsmittel, Treibmittel oder Pigmente.

Die Erfindung betrifft daher auch die Verwendung der Verbindungen der Formel I als Lichtschutzmittel für organische Materialien und die so stabilisierten Materialien, insbesondere polymeren Materialien. Die stabilisierten Polymeren können in verschiedener Form angewendet werden, z. B. als Folien, Fasern, Bändchen, Profile oder als Bindemittel für Lacke.

Die folgenden Beispiele beschreiben die Herstellung von Verbindungen der Formel I und deren Verwendung als Stabilisatoren.

## Beispiel 1

N-(2,2,6,6-Tetramethylpiperidin-4-yl)-tetrahydro-1,4-oxazin-2-on

Zu einer Mischung von 20 g (0,1 mol) 2-(2,2,6,6-Tetramethylpiperidin-4-ylamino)-ethanol und 21,2 g (0,2 mol) $Na_2CO_3$ werden unter Rühren innerhalb 3 h bei 120 °C 18 g (0,15 mol) Chloressigsäureethylester zugegeben. Nach einer weiteren Stunde Erwärmen wird das Reaktionsgemisch auf 25 °C abgekühlt und filtriert. Das Filtrat wird im Vakuum destilliert und man erhält 15,3 g der Titelverbindung, die bei 118-125 °C/0,1 mbar übergeht. Das Destillat erstarrt beim Stehen kristallin und schmilzt bei 77-85 °C.

Analyse : $C_{13}H_{24}N_2O_2$ (240,3)
ber. : C 65,0 H 10,1 N 11,7 %
gef. : C 65,0 H 10,1 W 11,7 %

NMR ($CDCl_3$) : $\delta = 1{,}03$ (t, 2H), 1,12 (s, 3H), 1,21 (s, 3H), 1,75 (dxd, 2H), 2,78 (Komplex, 3H), 3,47 (s, 2H), 4,36 (t, 2H).

Das JR-Spektrum zeigt eine starke Carbonylbande bei 1730 $cm^{-1}$.

## Beispiel 2

N-(2,2,6,6-Tetramethylpiperidin-4-yl)-tetrahydro-6-methyl-1,4-oxazin-2-on

Die Titelverbindung wird analog Beispiel 1 hergestellt unter Verwendung von 1-(2,2,6,6-Tetramethylpiperidin-4-ylamino)-propan-2-ol als Aminoalkohol. Die Verbindung siedet bei 121-127 °C/0,05 mbar.

Analyse : $C_{14}H_{26}N_2O_2$ (254,4)
ber. : C 66,1 H 10,3 N 11,0 %
gef. : C 65,6 H 10,6 N 10,8 %

NMR- und JR-Spektrum stimmen mit der angenommenen Struktur überein.

## Beispiel 3

N-(2,2,6,6-Tetramethylpiperidin-4-yl)-tetrahydro-3-phenyl-1,4-oxazin-2-on

Eine Mischung von 26,9 g (0,13 mol) 2-(2,2,6,6-Tetramethylpiperidin-4-ylamino)-ethanol, 30,8 g (0,13 mol) α-Bromphenylessigsäure-methylester und 14,2 g $Na_2CO_3$ in 100 ml Dimethylformamid wird unter Rühren 6 h auf 90 °C erwärmt. Das Lösungsmittel wird abdestilliert und der Rückstand zwischen 300 ml Diethylether und 100 ml Wasser verteilt. Die organische Phase wird mit 200 ml 1 n HCl extrahiert. Das saure Extrakt wird mit Natriumcarbonat neutralisiert und das Produkt in Chloroform aufgenommen. Die $CHCl_3$-Lösung wird eingedampft und der Rückstand aus Hexan kristallisiert. Man erhält 38 g farbloses Kristallisat, das bei 78-80 °C schmilzt.

Analyse : $C_{19}H_{29}N_2O_2$ (316,4)
ber. : C 72,1 H 8,9 N 8,8 %
gef. : C 72,2 H 8,9 N 8,9 %

## Beispiel 4

N-(2,2,6,6-Tetramethylpiperidin-4-yl)-tetrahydro-3-n-dodecyl-1,4-oxazin-2-on

Wenn in Beispiel 3 der Bromphenylessigsäure-Methylester durch die äquivalente Menge 2-Brommyristinsäure-ethylester ersetzt wird, so erhält man die Titelverbindung als viskoses gelbes Oel.
Analyse : $C_{25}H_{48}N_2O_2$ (408,7)
ber. : C 73,5 H 11,8 N 6,9 %
gef. : C 73,4 H 12,1 N 6,5 %

Beispiel 5

N-(2,2,6,6-Tetramethylpiperidin-4-yl)-tetrahydro-3-n-tetradecyl-1,4-oxazin-2-on

Analog Beispiel 4 wird bei Verwendung von 2-Brompalmitinsäure-ethylester die Titelverbindung als hellgelbe viskose Flüssigkeit erhalten.
Analyse : $C_{27}H_{52}N_2O_2$ (436,7)
ber. : C 74,1 H 12,0 N 6,4 %
gef. : C 73,5 H 12,1 N 6,2 %

Beispiel 6

Stabilisierung von Polypropylen-Bändchen

Polypropylen (Hercules Profax® 6501), das 0,1 % Ca-stearat aber kein Antioxydans enthält, wird mit 0,1 % Lichtschutzmittel vermischt, granuliert und in einem Extruder zu einem Band von 10,2 cm Breite und 0,12 mm Dicke verarbeitet (Düsentemperatur 232 °C). Das Band wird geschnitten in 6,4 mm breite Streifen, welche über Godet-Walzen bei 107 °C auf das sechsfache verstreckt werden. Die erhaltenen Bändchen haben eine Dicke von 0,05 mm.

Die Bändchen werden in einem Kohlebogen-Bewitterungsgerät belichtet. Von Zeit zu Zeit wird die Reissfestigkeit der belichteten Bändchen gemessen bis die Festigkeit auf die Hälfte abgesunken ist. Die Resultate sind in Tabelle 1 aufgeführt.

Tabelle 1

| Lichtschutzmittel (0,1 %) | Belichtungs-Stunden bis 50 % Reissfestigkeit |
|---|---|
| ohne | 230 |
| 2,4-Dihydroxybenzophenon | 240 |
| Verbindung des Beispiels 1 | 2 510 |

Beispiel 7

Stabilisierung von Polypropylen-Folien

Polypropylen (Hercules Profax® 6401) wird intensiv gemischt mit 0,2 % Di-octadecyl-3,5-di-t-butyl-4-hydroxybenzylphosphonat als Antioxydans und Verarbeitungsstabilisator und 0,25 % des in Tabelle 1 aufgeführten Lichtschutzmittels.

Das vermischte Material wird auf einem 2-Walzen Mischwerk bei 182 °C 5 min gewalzt. Die erhaltenen Walzfelle werden in einer hydraulischen Press bei 220 °C und 12,3 kg/cm$^2$ zu Filmen von 0,13 mm Dicke verpresst.

Die Filme werden mit einer Sonnenlicht-Fluoreszenzlampe belichtet und die Infrarot-Absorption der Filme bei 5,85 µm laufend gemessen bis die Absorption einen Wert von 0,5 erreicht hat. Die Belichtungszeit bis zu diesem Zeitpunkt ist ein Mass für die Wirkung des eingesetzten Lichtschutzmittels. Die Ergebnisse sind in Tabelle 2 aufgeführt.

Tabelle 2

| Lichtschutzmittel | Belichtungs-Stunden bis Absorption von 0,5 |
|---|---|
| ohne | 390 |
| Verbindung des Beispiels 2 | 1 270 |
| Verbindung des Beispiels 3 | 1 120 |

**Patentansprüche**

1. Verbindungen der Formel I

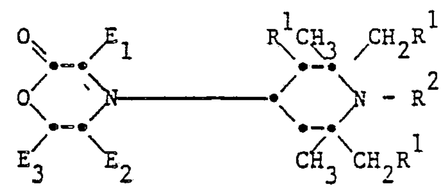

(I)

worin
$E_1$ Wasserstoff, $C_1$—$C_{16}$-Alkyl oder Phenyl bedeutet,
$E_2$ Wasserstoff oder Methyl bedeutet,
$E_3$ Wasserstoff, $C_1$—$C_4$-Alkyl oder Phenyl bedeutet,
$R^1$ Wasserstoff oder $C_1$—$C_5$-Alkyl bedeutet und
$R^2$ Wasserstoff, Sauerstoff, Hydroxyl, $C_1$—$C_{12}$-Alkyl, $C_3$—$C_8$-Alkenyl, Propargyl, Benzyl, Cyan, $C_2$—$C_4$-Hydroxyalkyl, $C_2$—$C_{10}$-Alkanoyl, $C_3$—$C_4$-Alkenoyl, Benzoyl, $C_1$—$C_8$-Alkoxy, $C_2$—$C_{11}$-Alkanoyloxy, $C_3$—$C_4$-Alkenoyloxy oder Benzoyloxy bedeutet.

2. Verbindungen gemäss Anspruch 1 der Formel I, worin $E_2$ Wasserstoff und $E_3$ Wasserstoff oder Methyl ist.

3. Verbindungen gemäss Anspruch 1 der Formel I, worin $R^1$ Wasserstoff ist.

4. Verbindungen gemäss Anspruch 1 der Formel I, worin $R^2$ Wasserstoff, Sauerstoff, Hydroxyl, $C_1$—$C_4$-Alkyl, Allyl, 2-Hydroxyethyl, Acetyl, Propargyl, Benzyl, Cyan, Benzoyl oder Benzoyloxy ist.

5. Verbindungen gemäss Anspruch 1 der Formel I, worin $R_2$ Wasserstoff ist.

6. Gegen Lichtschädigung stabilisiertes organisches Material enthaltend 0,05 bis 5 Gew.-% einer Verbindung gemäss Anspruch 1.

7. Stabilisiertes organisches Polymer gemäss Anspruch 6.

8. Stabilisiertes Polyolefin gemäss Anspruch 7.

9. Verwendung von Verbindungen des Anspruches 1 als Lichtschutzmittel in organischen Materialien, insbesondere in organischen Polymeren.

10. Verwendung gemäss Anspruch 9 in Polyolefinen.

**Claims**

1. A compound of the formula I

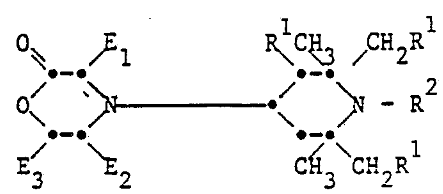

(I)

wherein
$E_1$ is hydrogen, $C_1$—$C_{16}$alkyl or phenyl,
$E_2$ is hydrogen or methyl
$E_3$ is hydrogen, $C_1$—$C_4$alkyl or phenyl,
$R^1$ is hydrogen or $C_1$—$C_5$alkyl, and
$R^2$ is hydrogen, oxygen, hydroxyl, $C_1$—$C_{12}$alkyl, $C_3$—$C_8$alkenyl, propargyl, benzyl, cyano, $C_2$—$C_4$hydroxyalkyl, $C_2$—$C_{10}$alkanoyl, $C_3$—$C_4$alkenoyl, benzoyl, $C_1$—$C_8$alkoxy, $C_2$—$C_{11}$alkanoyloxy, $C_3$—$C_4$alkenoyloxy or benzoyloxy.

2. A compound according to claim 1 of the formula I, wherein $E_2$ is hydrogen and $E_3$ is hydrogen or methyl.

3. A compound according to claim 1 of the formula I, wherein $R^1$ is hydrogen.

4. A compound according to claim 1 of the formula I, wherein $R^2$ is hydrogen, oxygen, hydroxyl, $C_1$—$C_4$alkyl, allyl, 2-hydroxyethyl, acetyl, propargyl, benzyl, cyano, benzoyl or benzoyloxy.

5. A compound according to claim 1 for the formula I, wherein $R^2$ is hydrogen.

6. An organic material stabilized against light-induced deterioration which contains 0,05 to 5 % by weight of a compound according claim 1.

7. A stabilized organic polymer according to claim 6.
8. A stabilized polyolefin according to claim 7.
9. Use of a compound of claim 1 as light stabilizer in organic materials, in particular in organic polymers.
10. Use according to claim 9, in polyolefins.

**Revendications**

1. Composés répondant à la formule I :

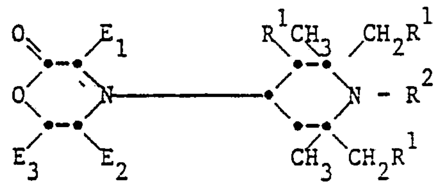

(I)

dans laquelle :

$E_1$ représente l'hydrogène, un alkyle en $C_1$—$C_{16}$ ou un phényle,
$E_2$ représente l'hydrogène ou un méthyle,
$E_3$ représente l'hydrogène, un alkyle en $C_1$—$C_4$ ou un phényle,
$R^1$ représente l'hydrogène ou un alkyle en $C_1$—$C_5$ et
$R^2$ représente l'hydrogène, l'oxygène, un hydroxy, un alkyle en $C_1$—$C_{12}$, un alcényle en $C_3$—$C_8$, un propyne-2 yle, un benzyle, un cyano, un hydroxyalkyle en $C_2$—$C_4$, un alcanoyle en $C_2$—$C_{10}$, un alcénoyle en $C_3$ ou $C_4$, un benzoyle, un alcoxy en $C_1$—$C_8$, un alcanoyloxy en $C_2$—$C_{11}$, un alcénoyloxy en $C_3$ ou $C_4$ ou un benzoyloxy.

2. Composés de formule I selon la revendication 1 dans lesquels $E_2$ représente l'hydrogène et $E_3$ l'hydrogène ou un méthyle.
3. Composés de formule I selon la revendication 1 dans lesquels $R^1$ représente l'hydrogène.
4. Composés de formule I selon la revendication 1 dans lesquels $R^2$ représente l'hydrogène, l'oxygène, un hydroxy, un alkyle en $C_1$—$C_4$, un allyle, un hydroxy-2 éthyle, un acétyle, un propyne-2 yle, un benzyle, un cyano, un benzoyle ou un benzoyloxy.
5. Composés de formule I selon la revendication 1 dans lesquels $R_2$ représente l'hydrogène.
6. Matière organique stabilisée contre la dégradation par la lumière, matière qui contient de 0,05 à 5 % en poids d'un composé selon la revendication 1.
7. Polymère organique stabilisé selon la revendication 6.
8. Polyoléfine stabilisée selon la revendication 7.
9. Application de composés selon la revendication 1 comme stabilisants à la lumière dans les matières organiques, plus spécialement dans des polymères organiques.
10. Application selon la revendication 9 dans des polyoléfines.